# EUROPEAN PATENT APPLICATION

(11) **EP 3 498 861 A1**
(43) Date of publication of application: **19.06.2019**
(21) Application number: 17819301.7
(22) Date of filing: 29.06.2017
(51) Int. Cl.: C12Q 1/68

(54) **RNA REVERSE TRANSCRIPTION AMPLIFICATION METHOD**

(30) Priority: 29.06.2016 CN 201610492890
(71) Applicant: Xiamen University, Xiamen, Fujian 361005 (CN)
(72) Inventor: ZHANG, Shiyin, Xiamen Fujian 361005 (CN); GE, Shengxiang, Xiamen Fujian 361005 (CN); WANG, Jin, Xiamen Fujian 361005 (CN); ZHANG, Jun, Xiamen Fujian 361005 (CN); XIA, Ningshao, Xiamen Fujian 361005 (CN)
(74) Representative: Held, Stephan
(86) International application number: PCT/CN2017/090759
(87) International publication number: WO 2018/001304

(57) **Abstract**

A ribonucleic acid (RNA) reverse transcription amplification method, comprising the steps of reverse transcription of RNA into cDNA and immediate amplification of cDNA, characterized in that the process of reverse transcription of RNA into cDNA is completed during the process that a cDNA amplification reaction system is heated to reach the reaction condition for cDNA amplification. The RNA reverse transcription amplification method can combine the reaction condition for reverse transcription of RNA into cDNA with the reaction condition for cDNA amplification, thereby significantly shorten the time required for RNA reverse transcription amplification. And in the whole process of RNA reverse transcription amplification, there is no need to change the instrument temperature, and thus, it is possible to achieve detection at any time.

## Description

### TECHNICAL FIELD

The present invention relates to a field of nucleic acid amplification, and particularly to a method for reverse transcription amplification of ribonucleic acid (RNA).

### BACKGROUND ART

As carriers of genetic information, DNA and RNA are important objects in life science research. However, DNA and RNA obtained directly from organisms are often insufficient in terms of load and need to be enriched by *in vitro* amplification. Currently, major methods for *in vitro* amplification of DNA include a polymerase chain reaction (PCR), isothermal amplification techniques (such as HDA, SDA, RPA, EXPAR, LAMP, NASBA, RCA, etc.) and the like. Most of such techniques use DNA as a direct amplification template.

*In vitro* amplification of RNA often requires synthesizing cDNA by using RNA as a template and using a reverse transcriptase at first, and then performing amplification with the resultant cDNA as a template. Compared with DNA amplification systems, RNA amplification systems have three different features: RNA, instead of DNA, is used as a template; a reverse transcriptase is needed to be added to the system; and an additional reverse transcription procedure is required before PCR amplification. Therefore, the efficiency of reverse transcription is mainly affected by the above three factors.
(1) Effect of RNA template on reverse transcription: a. Purity: High quality of RNA is a prerequisite for high reverse transcription efficiency, which can increase the length and yield of cDNA synthesis. On one hand, it is necessary to remove as many substances as possible, which may inhibit activities of reverse transcriptase and polymerase, such as lysis buffer, from a sample and extraction reagents; on the other hand, it is necessary to maintain RNA integrity as much as possible to avoid RNA fragmentation or degradation, for example, by adding an RNase inhibitor upon extraction. b. Structure: The secondary structure of RNA itself may affect the efficiency with which the primer binds to it, thereby further affecting the reverse transcription efficiency. Therefore, appropriately increasing the reverse transcription temperature, or adding an appropriate amount of DMSO or glycerol and the like to the system, has an auxiliary effect on unfolding the secondary structure of RNA, thereby improving the reverse transcription efficiency.
(2) Effects of enzymes on reverse transcription: a. Types of reverse transcriptase: The most commonly used reverse transcriptases include a murine leukemia reverse transcriptase (MMLV) and an avian myeloblastosis virus reverse transcriptase (AMV). The optimal reaction temperature of MMLV is 37 °C, and the length of the resultant synthetic cDNA approximately ranges from 100 nucleotides to 500 nucleotides. The optimal reaction temperature of AMV is 42°C to 55 °C, up to 60 °C. Therefore, if the RNA structure is more complicated, the application of AMV which can withstand higher temperature has a better effect in reverse transcription than that of MMLV, and in addition, the length range of cDNA synthesized *in vitro* under AMV is also better than under MMLV. However, the above two enzymes have RNase H activity in addition to DNA polymerase activity to catalyze the polymerization of dNTPs into cDNA with RNA as a template; In general, the RNaseH activity of AMV is a little higher than that of MMLV. The RNaseH activity may compete with the polymerase activity of reverse transcriptase for forming hybrid strand between a RNA template and DNA primers or cDNA extended strands, and degrade RNA strand in an RNA:DNA complex. RNA template degraded by RNaseH activity can no longer be used as an effective substrate for cDNA synthesis, and thus, the yield and length of cDNA synthesis are reduced. Therefore, most of the currently used commercial reverse transcriptases may be modified by reducing or removing their RNaseH activity by the way of mutation or deletion and the like. In addition, researchers may improve heat resistance of reverse transcriptase and speed of cDNA synthesis through genetic engineering, thereby improving reverse transcription efficiency. b. The amount of reverse transcriptase: When certain reverse transcription time is given, the reduced concentration of reverse transcriptase may lead to a decrease in the speed of cDNA synthesis. Therefore, appropriately increasing the concentration of the reverse transcriptase can increase the rate of cDNA synthesis per unit time, and to some extent, can make up for shortcomings of insufficient reverse transcription time.
(3) Effect of the program on reverse transcription: According to whether the cDNA synthesis process and the DNA amplification process are carried out in the same buffer and with the same enzyme, the amplification with RNA as a template can be classified as a two-step method and a one-step method.

In the two-step method, cDNA synthesis with RNA as a template is firstly performed in a reaction tube, and a small amount of the product in the tube is taken out as a template to carry out the second step of polymerase chain reaction. The advantages thereof are: reverse transcription and amplification are carried out in different reaction tubes, and conditions can be separately optimized. The disadvantages thereof are: the operation is complicated, sampling by opening the tube would easily cause contamination; only a portion of synthesized cDNA is used as a template to participate in the second round of amplification.

In the one-step method, cDNA synthesis and amplification with cDNA as a template are carried out in the same reaction tube. The advantages thereof are: (1) During the process of reverse transcription and amplification, it is not necessary to change the reaction tube, the operation is simple, and the contamination caused by the opening of the tube can be avoided; (2) all cDNAs synthesized by reverse transcription can be used as a starting template for PCR amplification. The one-step method was not popular at the time of its appearance, because its system optimization was more complicated than the two-step method. Specifically, all the reaction components in the one-step method are present in the same reaction tube, and the buffer conditions, ionic concentration, and the like in the reaction tube are required to satisfy both the requirements of reverse transcription and the requirements of subsequent amplification. In response to this problem, there are various commercial one-step RT-PCR reagent premixes on the market, which can not only ensure the reverse transcription efficiency, but also ensure the subsequent amplification in high efficiency. Researchers only need to add an appropriate amount of template, primers and water to the premixed liquid to complete the preparation of the reaction solution. Therefore, one-step RT-PCR, especially in the field of diagnosis, has been more and more widely recognized and applied.

Although the one-step method has been somewhat improved in terms of the operation step and the avoidance of contamination caused by the opening of the tube in comparison with the two-step method, a temperature-controlling program different from the subsequent amplification is still required. For methods of rapid detection of nucleic acids, especially amplification by a constant temperature method (such as an isothermal amplification, a convection PCR), the above disadvantage may make it impossible to achieve the all-process constant temperature from the initiation to the end of the reaction when performing the amplification with RNA as a template. Then, a program controlling module and a temperature controlling module having higher requirements have to be introduced into the constant temperature amplification device which is originally very simple. This will undoubtedly increase the weight, cost and operational complexity of the instrument to a certain extent; and it reduces the advantage of applying the constant temperature method for amplification.

### SUMMARY OF INVENTION

For the purpose of capable of improving the one-step RT-PCR, simplifying the instrument, and reducing the cost and the complexity of operation, the inventors, through repeated explorations and numerous experiments, have surprisingly found that the reverse transcription of RNA can be accomplished in the initial heating phase of the cDNA amplification process without the need for a separate process for reverse transcription of RNA into cDNA. The method greatly shortens the time of RT-PCR, simplifies the procedure, and finally achieves detection at any time, thereby completing the present invention.

The invention provides a RNA reverse transcription amplification method comprising the steps of reverse transcription of RNA into cDNA and immediate amplification of cDNA, characterized in that the process of the reverse transcription of RNA into cDNA is completed under reaction conditions for amplification of cDNA.

It is well known in the art that a one-step RNA reverse transcription amplification method generally comprises the steps of reverse transcription of RNA into cDNA and of immediate amplification of cDNA, and usually the two steps are separated in the program setup, namely, the program of the reverse transcription of RNA into cDNA is firstly set, for example, at 60 °C for 20 min, and then the step of amplifying the cDNA, for example, by employing a polymerase chain reaction or an isothermal amplification, is initiated. However, in the present invention, it is not necessary to separately set a program for reverse transcription of RNA into cDNA, and it is only necessary to complete the process of reverse transcription of RNA into cDNA under reaction conditions for amplification of cDNA.

In one embodiment of the present invention, the reaction system in the RNA reverse transcription amplification method is the same as the one-step RT-PCR system, that is, it comprises a reverse transcriptase, a DNA polymerase, dNTPs, a buffer, primers, and the like.

In one embodiment of the present invention, completing the process of reverse transcription of RNA into cDNA under reaction conditions for amplification of cDNA as described therein means that in the initial stage of the cDNA amplification reaction, i.e., during the process in which the cDNA amplification reaction system is heated to reach the reaction conditions for cDNA amplification, the process of reverse transcription of RNA into cDNA is completed.

It is well known in the art that in the initial stage of amplification of cDNA, a heating process is usually required to bring the reaction system to a certain temperature so as to allow the cDNA to be amplified in a denatured, single-stranded state. In the present invention, the step of reverse transcription of RNA into cDNA is completed by taking advantage of the heating process.

In one embodiment of the present invention, said heating to reach reaction conditions for cDNA amplification as described therein means that the temperature is raised from 0 °C or above, for example, from 4 °C or above, for example, raised from room temperature.

It is well known in the art that the optimal reaction temperature of some reverse transcriptases is about 37 °C (MMLV) or 42°C to 55 °C (AMV), so the initial temperature when heating needs to be lower than the optimal reaction temperature of the reverse transcriptase so as to complete the reverse transcription during the heating process.

In one embodiment of the present invention, said heating to reach reaction conditions for cDNA amplification as described therein means that the temperature is raised to a thermal denaturation temperature for cDNA amplification.

It is well known in the art that the initial stage of the reaction of cDNA amplification usually requires denaturing the cDNA to be amplified to be single-stranded and then performing amplification. Therefore, in the present invention, the step of reverse transcription of RNA into cDNA is a process in which the reverse transcription is performed during the stage where the initial temperature of cDNA amplification reaction is raised to the thermal denaturation temperature.

In one embodiment of the present invention, the method of amplifying cDNA described therein is a polymerase chain reaction or an isothermal amplification technique.

In one embodiment of the present invention, the polymerase chain reaction comprises a classical polymerase chain reaction (PCR), a convective PCR, a fluorescent quantitative PCR, and the like. In one embodiment of the present invention, the isothermal amplification technique comprises a rolling circle nucleic acid amplification (RCA), a helicase dependent DNA amplification, a nucleic acid sequence dependent amplification, a loop-mediated isothermal amplification, a single primer isothermal amplification, a rapid isothermal detection and amplification technique, a Qβ replication technique, a strand displacement amplification (SDA), a ligase chain reaction (LCR), and a nucleic acid sequence dependent amplification (NASBA) and the like.

In one embodiment of the present invention, the polymerase chain reaction described therein may be further divided into a polymerase chain reaction performed by thermal cycles of repeated heating and cooling with a heating module or a polymerase chain reaction performed by thermal convection.

In the present invention, the thermal denaturation temperature refers to a temperature required to break a hydrogen bond in double-stranded DNA and form two single-stranded DNA. In one embodiment of the present invention, the thermal denaturation temperature described therein refers to a temperature equal to or more than 90 °C, for example, 91 °C, 92 °C, 93 °C, 94 °C, 95 °C, 96 °C, 97 °C, 98 °C or 99 °C, depending on the length and structure of double-stranded DNA.

In one embodiment of the present invention, the heating rate described therein is about 2.0 °C to 4.5 °C/sec, for example, less than or equal to 4.4 °C/sec, or less than or equal to 3.5 °C/sec.

In one embodiment of the present invention, the reaction system comprises a reverse transcriptase, preferably, the reverse transcriptase is selected from the group consisting of a murine leukemia reverse transcriptase (MMLV) and an avian myeloblastosis virus reverse transcriptase (AMV). In a preferred embodiment of the invention, said DNA polymerase is a thermostable DNA polymerase.

In one embodiment of the present invention, the amount of the reverse transcriptase used in the process of reverse transcription of RNA into cDNA is greater than the amount of the reverse transcriptase when reaction conditions for reverse transcription of RNA into cDNA are separately set. In one embodiment of the present invention, when the heating rate is about 2.0 °C to 4.5 °C/sec, for example, less than or equal to 3.5 °C/sec, the effect of reverse transcription is generally not affected; if the effect of RNA reverse transcription during the heating process under certain conditions for cDNA amplification is lower than expectation, the reverse transcription effect can be enhanced by increasing the amount of the reverse transcriptase. In one embodiment of the present invention, the amount of the reverse transcriptase is increased by more than 10%, for example, more than 20%, 30%, 40%, 50%, 60%, 70%, 80%, 90%, 100%, 110%, 120%, 130%, 140% or 150%, with respect to the amount of the reverse transcriptase when the reaction conditions for reverse transcription of RNA into cDNA are separately set. In a specific embodiment of the invention, the amount of the reverse transcriptase is increased by 100%. In one embodiment of the present invention, the reaction conditions separately set for reverse transcription of RNA into cDNA are well known in the art, for example, at 60 °C for 20 min.

In one embodiment of the present invention, the process of reverse transcription of RNA into cDNA is terminated when the reverse transcriptase is inactivated as the temperature increases during the heating process.

In one embodiment of the present invention, the RNA fragment is less than 1000 bp in length, for example less than 900 bp, less than 800 bp, or less than 700 bp in length.

### Advantageous effects of invention

The RNA reverse transcription amplification method of the present invention can combine the reaction conditions for reverse transcription of RNA into cDNA and the reaction conditions for cDNA amplification, that is, after placing reaction tubes in the instrument, during the process where heating starts, temperature exchanges between reagents in the reaction tubes and heating parts and a temperature equilibration is finally achieved, the cDNA synthesis with RNA as a template is accomplished. The method significantly shortens the duration of RNA reverse transcription amplification, and makes it unnecessary to change the temperature of the instrument during the whole process of RNA reverse transcription amplification, so that detections can be achieved at any time. That is, the amplification of a sample is only related to the reaction time under a constant temperature, and there is no need for heating and cooling programs. Therefore, the temperature of the instrument can be fixed, and a new detection tube can be placed in the instrument for amplification at any time and the reaction tube can be taken out after a certain time to terminate the amplification.

### DESCRIPTION OF DRAWINGS

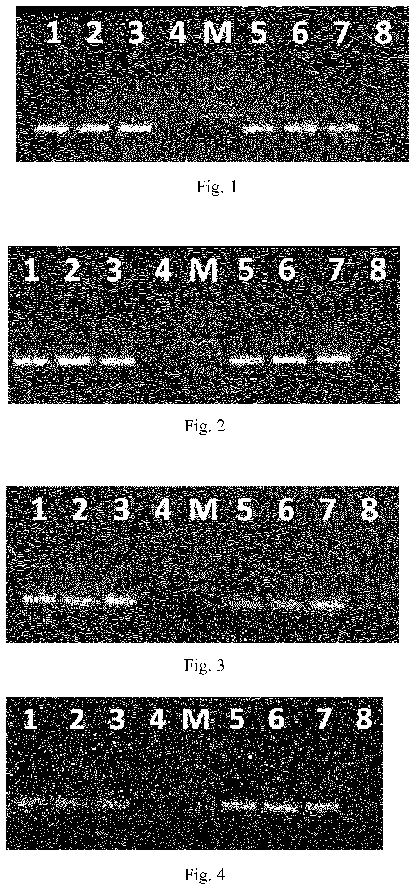

Figure 1: the agarose gel electrophoresis detection results of the products which are amplified in a convective PCR by using the present invention for rapid reverse transcription with EV71 virus (enteric virus type 71) single-stranded RNA as a template and then directly performing convective PCR amplification in the same tube; and the agarose gel electrophoresis detection results of the products amplified by firstly performing reverse transcription for 20 minutes with EV71 virus single-stranded RNA as a template and then directly performing convective PCR amplification in the same tube;
Figure 2: the agarose gel electrophoresis detection results of the products which are amplified in a convective PCR by using the present invention for rapid reverse transcription with RV virus (Rotavirus) double-stranded RNA as a template and then directly performing one-step convective PCR amplification in the same tube; and the agarose gel electrophoresis detection results of the products amplified by firstly performing reverse transcription for 20 minutes with RV virus double-stranded RNA as a template and then directly performing convective PCR amplification in the same tube;
Figure 3: the agarose gel electrophoresis detection results of the products which are amplified in a traditional PCR by using the present invention for rapid reverse transcription with EV71 virus single-stranded RNA as a template and then directly performing PCR amplification in the same tube; and the agarose gel electrophoresis detection results of the products amplified by firstly performing reverse transcription for 20 minutes with EV71 virus single-stranded RNA as a template and then directly performing PCR amplification in the same tube;
Figure 4: the agarose gel electrophoresis detection results of the products which are amplified in a helicase-dependent amplification (HDA) by using the present invention for rapid reverse transcription with EV71 virus single-stranded RNA as a template and then directly performing HDA amplification in the same tube; and the agarose gel electrophoresis detection results of the products amplified by firstly performing reverse transcription for 20 minutes with EV71 virus single-stranded RNA as a template and then performing HDA amplification in the same tube;

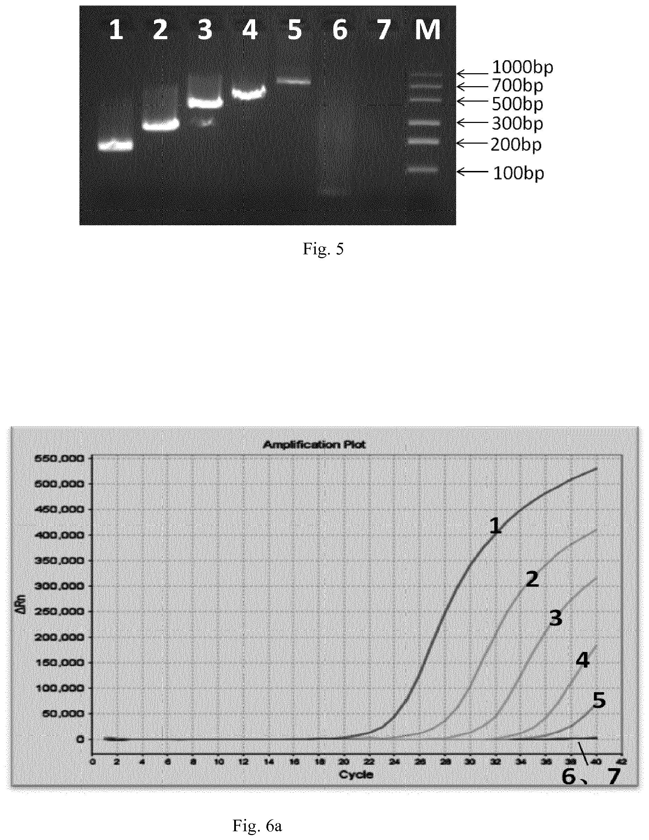

Figure 5: the agarose gel electrophoresis detection results of the products which are amplified in a convective PCR by using the present invention for rapid reverse transcription with EV71 virus single-stranded RNA as a template for fragments of different amplification lengths and then directly performing convective PCR in the same tube;
Figure 6a: the detection results of a fluorescent quantitative PCR in which a reverse transcription for 20 minutes with different concentrations of EV71 virus single-stranded RNAs as templates is firstly performed and then the fluorescent quantitative PCR is directly performed in the same tube;

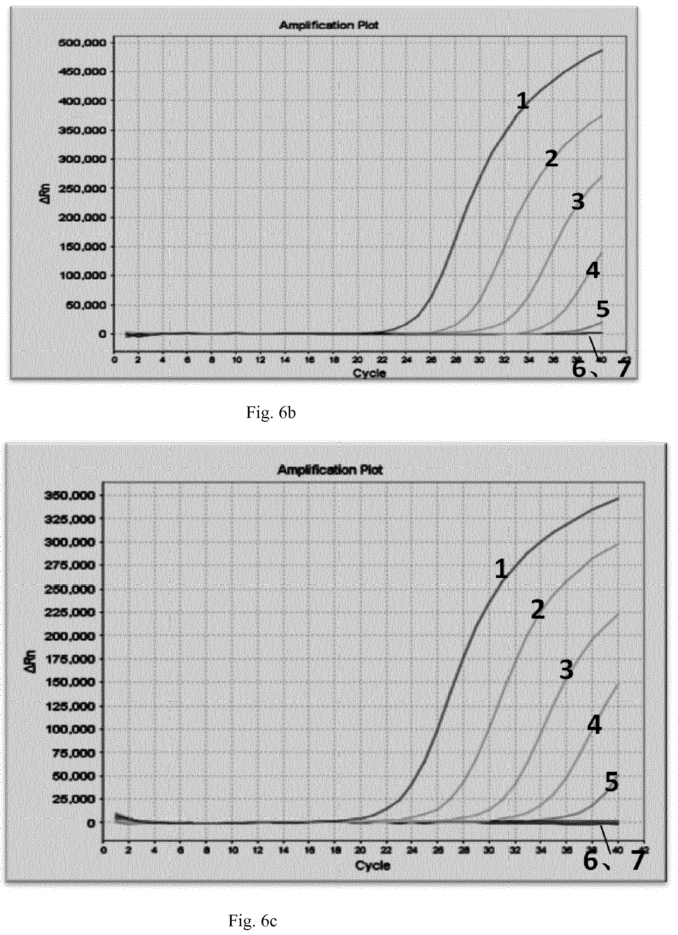

Figure 6b: the detection results of a fluorescent quantitative PCR in which the present invention is used for rapid reverse transcription with different concentrations of EV71 virus single-stranded RNAs as templates (relative to the detection as shown in Figure 6a, the amount of the reverse transcriptase is not varied) and then the fluorescent quantitative PCR is directly performed in the same tube;
Figure 6c: the detection results of a fluorescent quantitative PCR in which the present invention is used for rapid reverse transcription with different concentrations of EV71 virus single-stranded RNAs as templates (relative to the detection as shown in Figure 6a, the amount of reverse transcriptase is doubled) and then the fluorescent quantitative PCR is directly performed in the same tube.

### SEQUENCE LISTING

The specification of the present application includes a sequence listing, wherein the description of each sequence is as follows:
SEQ ID NO:1 primer 71FQ9F12
SEQ ID NO:2 primer 71FQ9R112
SEQ ID NO:3 primer NSP5-F1
SEQ ID NO:4 primer NSP5-R1
SEQ ID NO:5 primer CA16-F22
SEQ ID NO:6 primer CA16-R21
SEQ ID NO:7 primer CA16-F7
SEQ ID NO:8 primer CA16-F4
SEQ ID NO:9 primer CA16-F1
SEQ ID NO:10 primer CA16-F3
SEQ ID NO:11 primer CA16-F5
SEQ ID NO:12 probe 71FQ9P1

### EXAMPLES:

The embodiments of the present invention will be described in detail below with reference to accompanying examples. However, those skilled in the art will understand that, the following examples are only intended to illustrate the invention and are not to be construed as limiting the scope of the invention. For those without specific conditions specified in the examples, they are carried out according to general conditions or conditions recommended by the manufacturers. The reagents or instruments that are not specified in terms of the manufacturer are commercially available conventional products.

A method for performing a rapid RNA reverse transcription through a heat conduction process followed by an immediate nucleic acid amplification with cDNA as a template, comprises: in an optional nucleic acid amplification mode, during heat conduction between a heating module and reaction reagents, rapidly completing the RNA reverse transcription, and improving the efficiency of RNA reverse transcription by controlling the heating rate of the heating module, and(/or) increasing the amount of a reverse transcriptase; and immediately performing a method for nucleic acid amplification with cDNA synthesized by the reverse transcription as a template.

The present invention will be further described in detail below through the accompanying drawings and examples:
Fig. 1 shows the electrophoresis detection results of the products which are amplified in a convection PCR by using the rapid reverse transcription of the present invention and a common reverse transcription method, respectively, for transcription of EV71 virus single-stranded RNA and then performing one-step amplification. When applied, the reaction tube contains: a RNA template to be detected, a reverse transcriptase, a DNA polymerase, adenine deoxynucleotide triphosphate, cytosine deoxynucleotide triphosphate, guanine deoxynucleotide triphosphate, thymidine deoxynucleotide triphosphate, a reaction buffer, divalent magnesium ions, PCR additives which are not main components (such as betaine, bovine serum albumin, DMSO, etc.) and at least two oligonucleotide primers specifically complementary to the nucleic acid sequence to be detected. Moreover, the reagents' surface is covered with a low-density non-volatile substance (such as paraffin oil or various low-melting waxes) or the reaction tube is covered with a lid to prevent evaporation. In amplification, the reaction tubes are placed in a heating device (for the device, see the following patents for invention: CN103173434A, CN1571849A, and CN101983236A), the heating module at the bottom of the reaction tubes is set to 95 °C, the heating module at upper portion of the reaction tubes is set to 60 °C, and the reaction time is set to 30 minutes. After the reaction tubes are inserted into the instrument, the upper and lower metal blocks closely surrounding the reaction tubes transfer heat to the reaction liquid at corresponding portions in the tubes through the wall of the reaction tubes via heat conduction; meanwhile, the reagents in the upper and lower heated regions within the tubes transfer heat to reagent regions whose surrounding wall is not in contact with the metal blocks via heat conduction. Such heat transfer will allow a continuous temperature gradient to be formed in the reaction tube. Driven by the difference, the reagents in the tube will spontaneously flow and further transfer heat as the flow flows. In this process, when the reagents in the tubes are directly heated to reach the temperature range required by the reverse transcriptase, and when the reagents in the tubes pass through suitable temperature regions in the flow, cDNA is synthesized with RNA as a template under the action of the reverse transcriptase. When both heat transfer and heat convection reach a relatively stable state, the reverse transcriptase loses its activity when passing through the higher temperature region at the bottom portion. The reverse transcription process is terminated, and a convection PCR reaction is initiated with cDNA as a template in the reaction tubes. The results shown in Fig. 1 demonstrate that in convection PCR, the application of the rapid reverse transcription method of the present invention with the single-stranded RNA as a template can achieve the same effect as the conventional reverse transcription procedure.

Fig. 2 shows the electrophoresis detection results of the products which are amplified in a convection PCR by using the rapid reverse transcription of the present invention and a common reverse transcription method, respectively, for transcription of RV virus double-stranded RNA and then performing one-step amplification. The operation steps and reaction principle are identical to those for the single-stranded RNA described above, with the only difference being that the template is a double-stranded RNA. The results shown in Fig. 2 demonstrate that in convection PCR, the application of the rapid reverse transcription method of the present invention with the double-stranded RNA as a template also can achieve the same effect as the conventional reverse transcription procedure.

Fig. 3 shows the electrophoresis detection results of the products which are amplified in a traditional PCR by using the rapid reverse transcription of the present invention and a common reverse transcription method, respectively, for transcription of EV71 virus RNA and then performing one-step amplification. Unlike the rapid reverse transcription using the convective PCR conditions, there are no temperature gradient within the tube and portions that undergoes reverse transcription in appropriate temperature regions in the gradient in the case of the traditional PCR. In the traditional PCR, after the reaction tubes are placed in the instrument and the program is initiated, the metal heating module of the instrument will transfer heat to the reaction tubes while raising the temperature, until both the metal heating module of the instrument and the reagents in the tubes reach the pre-set temperature. During this period, the temperature of the reagents in the tubes continuously increases with time. In this process, when the temperature interval in a specific time range exactly satisfies working conditions for the reverse transcription, cDNA will be synthesized with RNA as a template under the action of reverse transcriptase. The results shown in Fig. 3 demonstrate that in traditional PCR, the application of the rapid reverse transcription method of the present invention can achieve the same effect as the conventional reverse transcription procedure.

Fig. 4 shows the electrophoresis detection results of the products of the isothermal amplification exemplified by HDA by using the rapid reverse transcription of the present invention and a common reverse transcription method, respectively, for transcription of EV71 virus RNA and then performing one-step amplification. When applied, the reaction tube contains: a RNA template to be detected, a Bst polymerase, a UvrD helicase, adenine deoxynucleotide triphosphate, cytosine deoxynucleotide triphosphate, guanine deoxynucleotide triphosphate, thymidine deoxynucleotide triphosphate, a reaction buffer, divalent magnesium ions, PCR additives which are not main components (such as betaine, bovine serum albumin, DMSO, etc.) and at least two oligonucleotide primers specifically complementary to the nucleic acid sequence to be detected. Moreover, the reagents' surface is covered with a low-density non-volatile substance (such as paraffin oil or various low-melting waxes) or the reaction tube is covered with a lid to prevent evaporation. Its rapid reverse transcription process and principle are consistent with the traditional PCR described above. The results shown in Fig. 4 demonstrate that in the isothermal amplification exemplified by HDA, the application of the rapid reverse transcription method of the present invention can achieve the same effect as the conventional reverse transcription procedure.

Fig. 5 shows the electrophoresis detection results of the products of the convection PCR by using the rapid reverse transcription of the present invention for transcription of EV71 virus single-stranded RNA and then performing amplification of fragments in different lengths. The results shown in this figure demonstrate that the reverse transcription in a length of 649 bp can be achieved by using the rapid reverse transcription method of the present invention.

Fig. 6a shows the real-time detection curve of the sample produced by firstly performing reverse transcription for 20 min for single-stranded RNA templates with different concentrations and then performing one-step amplification; Fig. 6b shows the real-time detection curve of the sample produced by using the present invention for rapid reverse transcription of single-stranded RNA templates with different concentrations and then directly performing one-step amplification. The rapid reverse transcription method has a somewhat delayed Ct value as compared with the method of first reverse transcription followed by amplification for 20 min; Fig. 6c shows the real-time detection curve of the sample produced by using the present invention for rapid reverse transcription of single-stranded RNA templates with different concentrations in the presence of a double amount of reverse transcriptase and then directly performing one-step amplification. The results shown in this figure demonstrate that the delay of the Ct value caused by the rapid reverse transcription method can be compensated by increasing the amount of reverse transcriptase.

Unless otherwise specified, the molecular biology experimental methods and immunoassays used in the present invention are carried out essentially according to the methods described in J. Sambrook et al., Molecular Cloning: A Laboratory Manual, 2nd Edition, Cold Spring Harbor Laboratory Press, 1989, and F. M. Ausubel et al., Current Protocols in Molecular Biology, 3rd Edition, John Wiley & Sons, Inc., 1995; enzymes are used according to the conditions recommended by the product manufacturers. Those skilled in the art are aware that the examples are used to illustrate the present invention by way of example, and are not intended to limit the scope of the invention.

### Example 1: The amplification and detection of the products produced by using the present invention for rapid reverse transcription with EV71 virus single-stranded RNA as a template in convective PCR

### 1. Experimental materials

Chemical reagents: SpeedSTAR HS DNA polymerase (TaKaRa), reverse transcriptase MMLV (Transgen), 10 x Fast Buffer I (Mg²⁺ plus) (TaKaRa), dNTPs (TaKaRa), DEPC water, paraffin oil, and 6 x DNA loading Buffer (containing Sybr Green).

Instruments and consumable materials: A self-made nucleic acid amplification instrument (see Figure 2 of Chinese Patent Application No. 201110456811.9), which has an upper temperature-controlling average heating rate of 20.8 °C/min, and a lower temperature-controlling average heating rate of 29.05 °C/min; self-made nucleic acid amplification reaction tubes (see Example 1 of Chinese Patent No. ZL201110360350.5), a gel electrophoresis apparatus, and a gel imager (Bio-Rad).
Primers:
71FQ9F12: GYTTCRGTGCCATTCATgTCAC (SEQ ID NO:1)
71FQ9R112: GCCCCATATTCAAGRTCTTTCTC (SEQ ID NO:2)

All the detection templates 1-3 and 5-7 are EV71 viral RNA extract with a concentration of 10⁴ copies/mL.

Detection templates 4 and 8 are DEPC water.

### 2. Experimental methods

(1) Preparation of amplification reagent: The reaction solution is prepared in an ice bath according to the following formula: 3.2 mM dNTPs, 4 µL 10 x Fast Buffer I (Mg²⁺ plus), 1 U SpeedSTAR HS DNA polymerase, 16U MMLV, 0.4 µL 10 µM 71FQ9F12, 0.4 µL 10 µM 71FQ9R112. 5 µl of one detection template is added to each tube, the total volume is 40 µl, and the remaining volume is balanced with DEPC water.
(2) Nucleic acid amplification: a. The prepared amplification reagent is injected into a nucleic acid amplification reaction tube, into which 10 µl paraffin oil is dropwise added, and the reaction container is filled with the amplification reagent by centrifugation, agitation or other means, and stored in an ice bath; b1. The rapid reverse transcription method of the invention is applied: the bottom heating module of the instrument is set to a constant temperature of 95 °C, and the upper heating module of the instrument is set to a constant temperature of 60 °C. After the temperature is equilibrated, the reaction tube containing the nucleic acid amplification reagent is inserted into the instrument. The reaction tube is taken out after 30 minutes of reaction; b2. The common reverse transcription method is applied: both upper and bottom heating modules of the instrument are set to 60 °C. After the temperature is equilibrated, the reaction tube containing the nucleic acid amplification reagent is inserted into the instrument. After 20 minutes of reaction, the temperature of the bottom aluminum block is raised to 95 °C, and the temperature of the upper aluminum block remains unchanged. The reaction tube is taken out after 30 minutes of reaction.
(3) Electrophoresis detection of the amplification products: 5 µl of the amplification product is taken out of each reaction tube, mixed with 1 µl loading buffer, and thereafter the amplified products are detected by 3% agarose gel electrophoresis.
   3. Experimental results: As shown in Fig. 1, in the results of amplification of positive samples using the rapid reverse transcription of the present invention (lanes 1-3) and the results of amplification of positive samples using the common reverse transcription method (lanes 5-7), the amplified bands are uniform in terms of brightness and homogeneity, the background is clean, and there are no non-specific amplification products such as primer dimers and the like. In both the results of amplification of negative sample using the rapid reverse transcription of the present invention (lane 4) and the results of amplification of negative sample using the common reverse transcription method (lane 8), there is no non-specific amplification. The above results demonstrate that the rapid reverse transcription method of the present invention can accomplish the cDNA synthesis with single-stranded RNA as a template and the immediate convective amplification with the cDNA as a template in the convective PCR.

### Example 2: The amplification and detection of the products produced by using the present invention for rapid reverse transcription with RV virus double-stranded RNA as a template in convective PCR

### 1. Experimental materials

Chemical reagents: SpeedSTAR HS DNA polymerase (TaKaRa), reverse transcriptase MMLV (Transgen), 10 x Fast Buffer I (Mg²⁺ plus) (TaKaRa), dNTPs (TaKaRa), DEPC water, paraffin oil, and 6 x DNA loading Buffer (containing Sybr Green).

Instruments and consumable materials: A self-made nucleic acid amplification instrument (see Figure 2 of Chinese Patent Application No. 201110456811.9), which has an upper temperature-controlling average heating rate of 20.8 °C/min, and a lower temperature-controlling average heating rate of 29.05 °C/min; self-made nucleic acid amplification reaction tubes (see Example 1 of Chinese Patent No. ZL201110360350.5), a gel electrophoresis apparatus, and a gel imager (Bio-Rad).
Primers:
NSP5-F1: AGAGGATATTGGACCATCTGA (SEQ ID NO:3)
NSP5-R1: GAATCCATAGACACGCCAG (SEQ ID NO:4)

All the detection templates 1-3 and 5-7 are RV viral RNA extract with a concentration of 10⁴ copies/mL.

Detection templates 4 and 8 are DEPC water.

### 2. Experimental methods

(1) Preparation of amplification reagent: The reaction solution is prepared in an ice bath according to the following formula: 3.2 mM dNTPs, 4 µL 10× Fast Buffer I (Mg²⁺ plus), 1 U SpeedSTAR HS DNA polymerase, 16U MMLV, 0.4 µL 10 µM NSP5-F1, 0.4 µL 10 µM NSP5-R1. 5 µl of one detection template is added to each tube, the total volume is 40 µl, and the remaining volume is balanced with DEPC water.
(2) Nucleic acid amplification: a. The prepared amplification reagent is injected into a nucleic acid amplification reaction tube, into which 10 µl of paraffin oil is dropwise added, and the reaction container is filled with the amplification reagent by centrifugation, agitation or other means, and stored in an ice bath; b1. The rapid reverse transcription method of the invention is applied: the bottom heating module of the instrument is set to a constant temperature of 95 °C, and the upper heating module of the instrument is set to a constant temperature of 60 °C. After the temperature is equilibrated, the reaction tube containing the nucleic acid amplification reagent is inserted into the instrument. The reaction tube is taken out after 30 minutes of reaction; b2. The common reverse transcription method is applied: both upper and bottom heating modules of the instrument are set to 60 °C. After the temperature is equilibrated, the reaction tube containing the nucleic acid amplification reagent is inserted into the instrument. After 20 minutes of reaction, the temperature of the bottom aluminum block is raised to 95 °C, and the temperature of the upper aluminum block remains unchanged. The reaction tube is taken out after 30 minutes of reaction.
(3) Electrophoresis detection of the amplification products: 5 µl of the amplification product is taken out of each reaction tube, mixed with 1 µl loading buffer, and thereafter the amplified products are detected by 3% agarose gel electrophoresis.
   3. Experimental results: As shown in Fig. 2, in the results of amplification of positive samples using the rapid reverse transcription of the present invention (lanes 1-3) and the results of amplification of positive samples using the common reverse transcription method (lanes 5-7), the amplified bands are uniform in terms of brightness and homogeneity, the background is clean, and there are no non-specific amplification products such as primer dimers and the like. In both the results of amplification of negative sample using the rapid reverse transcription of the present invention (lane 4) and the results of amplification of negative sample using the common reverse transcription method (lane 8), there is no non-specific amplification. The above results demonstrate that the rapid reverse transcription method of the present invention can accomplish the cDNA synthesis with double-stranded RNA as a template and the immediate convective amplification with the cDNA as a template in the convective PCR.

### Example 3: The amplification and detection of the products produced by using the present invention for rapid reverse transcription with EV71 virus single-stranded RNA as a template in the traditional PCR

### 1. Experimental materials

Chemical reagents: SpeedSTAR HS DNA polymerase (TaKaRa), reverse transcriptase MMLV (Transgen), 10 x Fast Buffer I (Mg²⁺ plus) (TaKaRa), dNTPs (TaKaRa), DEPC water, paraffin oil, 6 x DNA loading Buffer (containing Sybr Green)
Instruments and consumable materials: A thermal cycler (Bio-Rad PTC0220), which has an average heating rate of 3.5 °C/s; PCR reaction tubes, a gel electrophoresis apparatus, and a gel imager (Bio-Rad).
Primers:
71FQ9F12: GYTTCRGTGCCATTCATgTCAC (SEQ ID NO:1)
71FQ9R112: GCCCCATATTCAAGRTCTTTCTC (SEQ ID NO:2)

All the detection templates 1-3 and 5-7 are EV71 viral RNA extract with a concentration of 10⁴ copies/mL.

Detection templates 4 and 8 are DEPC water.

### 2. Experimental methods

(1) Preparation of amplification reagent: The reaction solution is prepared in an ice bath according to the following formula: 1.6 mM dNTPs, 2 µL 10× Fast Buffer I (Mg2+ plus), 0.5 U SpeedSTAR HS DNA polymerase, 16U MMLV, 0.2 µL 10 µM 71FQ9F12, 0.2 µL 10 µM 71FQ9R112. 5 µl of one detection template is added to each tube, the total volume is 20 µl, and the remaining volume is balanced with DEPC water.
(2) Nucleic acid amplification: a. The PCR reaction tube containing the prepared amplification reagent and stored in the ice bath is placed in the reaction tank of the PCR instrument; b1. The rapid reverse transcription method of the present invention is applied: a thermal cycling program is set as follows: pre-denaturation at 95 °C for 5 min and then 40 cycles of (95 °C 20s - 60 °C 20s). The reaction tube is taken out after the completion of the reaction; b2. The common reverse transcription method is applied: reverse transcription at 60 °C for 20 min, pre-denaturation at 95 °C for 5 min, and then 40 cycles of (95 °C 20 s - 60 °C 20 s). The reaction tube is taken out after the completion of the reaction.
(3) Electrophoresis detection of the amplification products: 5 µl of the amplification product is taken out of each reaction tube, mixed with 1 µl loading buffer, and thereafter the amplified products are detected by 3% agarose gel electrophoresis.
   3. Experimental results: As shown in Fig. 3, in the results of amplification of positive samples using the rapid reverse transcription of the present invention (lanes 1-3) and the results of amplification of positive samples using the common reverse transcription method (lanes 5-7), the amplified bands are uniform in terms of brightness and homogeneity, the background is clean, and there are no non-specific amplification products such as primer dimers and the like. In both the results of amplification of negative sample using the rapid reverse transcription of the present invention (lane 4) and the results of amplification of negative sample using the common reverse transcription method (lane 8), there is no non-specific amplification. The above results demonstrate that the rapid reverse transcription method of the present invention can accomplish the cDNA synthesis with single-stranded RNA as a template and the immediate PCR amplification with the cDNA as a template in the traditional PCR.

### Example 4: The amplification and detection of the products produced by using the rapid reverse transcription method for cDNA synthesis with EV71 virus single-stranded RNA as a template and the immediate HDA

### 1. Experimental materials

Chemical reagents: Bst polymerase and UvrD helicase (NEB), reverse transcriptase MMLV (Transgen), 10 x Annealing Buffer I (Mg²⁺ plus) (NEB), dNTPs (TaKaRa), dATP (TaKaRa), DEPC water, and 6 x DNA Loading Buffer (containing Sybr Green).

Instruments and consumable materials: A thermal cycler (Bio-Rad PTC0220), which has an average heating rate of 3.5 °C/s; PCR reaction tubes, a gel electrophoresis apparatus, and a gel imager (Bio-Rad).
Primers:
71FQ9F12: GYTTCRGTGCCATTCATgTCAC (SEQ ID NO:1)
71FQ9R112: GCCCCATATTCAAGRTCTTTCTC (SEQ ID NO:2)

All the detection templates 1-3 and 5-7 are EV71 viral RNA extract with a concentration of 10⁴ copies/mL.

Detection templates 4 and 8 are DEPC water.

### 2. Experimental methods

(1) Preparation of amplification reagent: The reaction solution is prepared in an ice bath according to the following formula: 1.6 mM dNTPs, 1 mM dATP, 2 µL 10× Annealing Buffer (Mg²⁺ plus), Bst polymerase (1mg/ml) 0.3ul, UvrD helicase (0.7mg/ml) 2 µl, 16U MMLV, 0.2 µL 10 µM 71FQ9F12, 0.2 µL 10 µM 71FQ9R112. 5 µl of one detection template is added to each tube, the total volume is 20 µl, and the remaining volume is balanced with DEPC water.
(2) Nucleic acid amplification: a. The PCR reaction tubes containing the prepared amplification reagent and stored in the ice bath are placed in the reaction tank of the PCR instrument; b1. The rapid reverse transcription method of the invention is applied: a constant temperature of 65 °C and a reaction time of 30 min are set. The reaction tube is taken out after the completion of the reaction; b2. The common reverse transcription method is applied: after reverse transcription at 60 °C for 20 min, the temperature is raised to 65 °C to react for 30 min. The reaction tubes are taken out after the completion of the reaction.
(3) Electrophoresis detection of the amplification products: 5 µl of the amplification product is taken out of each reaction tube, mixed with 1 µl loading buffer, and thereafter the amplified products are detected by 3% agarose gel electrophoresis.
   3. Experimental results: As shown in Fig. 4, in the results of amplification of positive samples using the rapid reverse transcription of the present invention (lanes 1-3) and the results of amplification of positive samples using the common reverse transcription method (lanes 5-7), the amplified bands are uniform in terms of brightness and homogeneity, the background is clean, and there are no non-specific amplification products such as primer dimers and the like. In both the results of amplification of negative sample using the rapid reverse transcription of the present invention (lane 4) and the results of amplification of negative sample using the common reverse transcription method (lane 8), there is no non-specific amplification. The above results demonstrate that, in HAD, the rapid reverse transcription method of the present invention can accomplish the cDNA synthesis with single-stranded RNA as a template and the immediate HDA amplification with the cDNA as a template.

### Example 5: The detection of reverse transcription and amplification of fragments of different lengths by using the rapid reverse transcription method

### 1. Experimental materials

Chemical reagents: SpeedSTAR HS DNA polymerase (TaKaRa), reverse transcriptase MMLV (Transgen), 10 x Fast Buffer I (Mg²⁺ plus) (TaKaRa), dNTPs (TaKaRa), DEPC water, paraffin oil, and 6 x DNA loading Buffer (containing Sybr Green).

Instruments and consumable materials: A self-made nucleic acid amplification instrument (see Figure 2 of Chinese Patent Application No. 201110456811.9), which has an upper temperature-controlling average heating rate of 20.8 °C/min, and a lower temperature-controlling average heating rate of 29.05 °C/min; self-made nucleic acid amplification reaction tubes (see Example 1 of Chinese Patent No. ZL201110360350.5), a gel electrophoresis apparatus, and a gel imager (Bio-Rad).
Primer 1: the amplification length is 166 bp
   CA16-F22: CCGAATAATATGATGGGCACTTTTAG (SEQ ID NO:5)
   CA16-R21: CCTTTATAATTTGGGTTGGTCTTA (SEQ ID NO:6)
Primer 2: the amplification length is 283 bp
   CA16-F7: CCAGCTCAAGTGTCAGTCCC (SEQ ID NO:7)
   CA16-R21: CCTTTATAATTTGGGTTGGTCTTA (SEQ ID NO:6)
Primer 3: the amplification length is 455 bp
   CA16-F4 : CGCTTYGATGCTGAATTYAC (SEQ ID NO:8)
   CA16-R21: CCTTTATAATTTGGGTTGGTCTTA (SEQ ID NO:6)
Primer 4: the amplification length is 514 bp
   CA16-F1: GACATTGAYTTGATGGGATATGCTC (SEQ ID NO:9)
   CA16-R21: CCTTTATAATTTGGGTTGGTCTTA (SEQ ID NO:6)
Primer 5: the amplification length is 649 bp
   CA16-F3: GAGACKAGATGTGTGTTGAAYCA (SEQ ID NO:10)
   CA16-R21: CCTTTATAATTTGGGTTGGTCTTA (SEQ ID NO:6)
Primer 6: the amplification length is 835 bp
   CA16-F5: CATTGCAGAYATGATCGACCA (SEQ ID NO:11)
   CA16-R21: CCTTTATAATTTGGGTTGGTCTTA (SEQ ID NO:6)

Detection templates 1-6 are CA16 viral RNA extract with a concentration of 10⁴ copies/mL.

Detection template 7 is DEPC water.

### 2. Experimental methods

(1) Preparation of amplification reagent: The reaction solution is prepared in an ice bath according to the following formula: 3.2 mM dNTPs, 4 µL 10× Fast Buffer I (Mg²⁺ plus), 1 U SpeedSTAR HS DNA polymerase, 16U MMLV, 0.4 µL 10 µM upstream primer, 0.4 µL 10 µM downstream primer. 5 µl of one detection template is added to each tube, the total volume is 40 µl, and the remaining volume is balanced with DEPC water.
(2) Nucleic acid amplification: a. The prepared amplification reagent is injected into a nucleic acid amplification reaction tube, into which 10 µl paraffin oil is added, and the reaction container is filled with the amplification reagent by centrifugation, agitation or other means, and stored in an ice bath; b. the bottom heating module of the instrument is set to a constant temperature of 95 °C, and the upper heating module of the instrument is set to a constant temperature of 60 °C. After the temperature is equilibrated, the reaction tubes containing the nucleic acid amplification reagent are inserted into the instrument. The reaction tubes are taken out after 30 minutes of reaction.
(3) Electrophoresis detection of the amplification products: 5 µl of the amplification product is taken out of each reaction tube, mixed with 1 µl loading buffer, and thereafter the amplified products are detected by 3% agarose gel electrophoresis.
   3. Experimental results: As shown in Fig. 5, lanes 1-6 correspond to amplification of fragments of 166 bp, 283 bp, 455 bp, 514 bp, 649 bp, and 835 bp, respectively, and lane 7 is a negative control of primer 1. It can be seen from the above results that the rapid reverse transcription method of the present invention can accomplish the cDNA synthesis of fragments in different lengths and the immediate convective amplification with the cDNA as a template.

### Example 6: The detection of reverse transcription and amplification with different concentrations of RNA templates by using the rapid reverse transcription method

### 1. Experimental materials

Chemical reagents: SpeedSTAR HS DNA polymerase (TaKaRa), reverse transcriptase MMLV (Transgen), 10 x Fast Buffer I (Mg²⁺ plus) (TaKaRa), dNTPs (TaKaRa), DEPC water, paraffin oil, and 6 x DNA loading Buffer (containing Sybr Green).

Instruments and consumable materials: ABI7500, ABI 8-strip tubes.
Primers:
   71FQ9F12: GYTTCRGTGCCATTCATgTCAC (SEQ ID NO:1)
   71FQ9R112: GCCCCATATTCAAGRTCTTTCTC (SEQ ID NO:2)
Probe:
   71FQ9P1: TAYGACGGRTAYCCCACRTTYGGWGA (5' end is labeled with FAM, and 3' end is labeled with BHQ1) (SEQ ID NO:12)
   Detection templates 1-5 are EV71 viral RNA extract at a concentration of 10⁷ copies/ml, 10⁶ copies/ml, 10⁵ copies/ml, 10⁴ copies/ml, or 10³ copies/ml, respectively.

Detection templates 6-7 are DEPC water.

### 2. Experimental methods

(1) Preparation of amplification reagent: The reaction solution is prepared in an ice bath according to the following formula: 1.6 mM dNTPs, 2µL 10× Fast Buffer I (Mg²⁺ plus), 0.5 U HS Taq (TaKaRa), 16U MMLV (Groups a and b are 16U, Group c is 32U), 0.2 µL 10 µM 71FQ9F12, 0.2µL 10 µM 71FQ9R112, 0.1 µL 10 µM 71FQ9P1. 5 µl of one detection template is added to each tube, the total volume is 20 µl, and the remaining volume is balanced with DEPC water.
(2) Nucleic acid amplification: a. The PCR reaction tubes containing the prepared amplification reagent are placed in the reaction tank of the PCR instrument and stored in the ice bath; b1. The common reverse transcription: reverse transcription at 60 °C for 20 min, then pre-denaturation at 95 °C for 5 min, and 40 cycles of (95 °C 20 s - 60 °C 20 s); b2. The rapid reverse transcription: pre-denaturation at 95 °C for 5 min, and 40 cycles of (95 °C 20 s, 60 °C 20 s).
   3. Experimental results: In the fluorescent quantitative PCR, the Ct value represents the number of cycles experienced by the fluorescence signal in each tube when reaching a set threshold, wherein C represents Cycle (number of cycles) and t represents Threshold (threshold value). As shown in the data of Figure 6 and Table 1, without changing the amount of the reverse transcriptase, the Ct value of the sample which is directly subjected to one-step amplification after rapid reverse transcription of single-stranded RNA which is used as different concentrations of template in the method of the present invention (Fig. 6b, Group b in Table 1) is delayed relative to the Ct value of the sample which is firstly reverse-transcribed for 20 min and then amplified in one-step amplification (Fig. 6a, Group a in Table 1). When the amount of the reverse transcriptase is doubled, the Ct value of the sample which is directly subjected to one-step amplification after rapid reverse transcription of single-stranded RNA which is used as different concentrations of template in the method of the present invention (Fig. 6c, Group c in Table 1) can achieve the same reverse transcription and amplification effects as shown in Fig. 6a. The experimental results demonstrate that under the same conditions, the reverse transcription efficiency of the rapid reverse transcription method is slightly lower than that of the common reverse transcription method, but the decrease in efficiency can be compensated by appropriately increasing the amount of the reverse transcriptase, finally achieving the same detection effect.

**Table 1 Comparison of Ct values of samples amplified in different reverse transcription methods or conditions**

| Detection template | 1 | 2 | 3 | 4 | 5 | 6 | 7 |
|---|---|---|---|---|---|---|---|
| Group a | 21.52 | 25.66 | 29.57 | 33.74 | 36.44 | N | N |
| Group b | 22.66 | 26.84 | 29.85 | 34.45 | 38.02 | N | N |
| Group c | 21.27 | 25.25 | 29.3 | 32.71 | 36.75 | N | N |

| | | | | | | | |
|---|---|---|---|---|---|---|---|
| Note: wherein 1 represents 10⁷ copies/ml, 2 represents 10⁶ copies/ml, 3 represents 10⁵ copies/ml, 4 represents 10⁴ copies/ml, 5 represents 10³ copies/ml, and the detection templates 6 and 7 are DEPC water, representing negative controls. "N" indicates that the fluorescent quantitative PCR detection result is negative. | | | | | | | |

### Example 7: The detection of reverse transcription and amplification of RNA templates by using the rapid reverse transcription method under different heating rates

### 1. Experimental materials

Chemical reagents: SpeedSTAR HS DNA polymerase (TaKaRa), reverse transcriptase MMLV (Transgen), 10 x Fast Buffer I (Mg²⁺ plus) (TaKaRa), dNTPs (TaKaRa), DEPC water, paraffin oil, 6 x DNA loading Buffer (containing Sybr Green).

Instruments and consumable materials: A Fluorescence quantitative PCR instrument (Roche LightCycler 96); PCR reaction tubes.
Primers:
   71FQ9F12: GYTTCRGTGCCATTCATgTCAC (SEQ ID NO:1)
   71FQ9R112: GCCCCATATTCAAGRTCTTTCTC (SEQ ID NO:2)
Probe:
   71FQ9P1: TAYGACGGRTAYCCCACRTTYGGWGA (5' end is labeled with FAM, and 3' end is labeled with BHQ1) (SEQ ID NO:12)
   Detection templates 1-4 are EV71 viral RNA extract at a concentration of 10⁵ copies/ml, 10⁴ copies/ml, 10³ copies/ml, or 10² copies/ml, respectively.

Detection templates 5-6 are DEPC water.

### 2. Experimental methods

(1) Preparation of amplification reagent:
   The reaction solution is prepared in an ice bath according to the following formula: 1.6 mM dNTP, 2 µL 10× Fast Buffer I (Mg²⁺ plus), 0.5 U HS Taq (TaKaRa), MMLV (16U in Groups a, b, c, and d; 32U in Group e), 0.2 µL 10 µM 71FQ9F12, 0.2 µL 10 µM 71FQ9R112, 0.1 µL 10 µM 71FQ9P1. 5 µl of one detection template is added to each tube, the total volume is 20 µl, and the remaining volume is balanced with DEPC water.
(2) a. The PCR reaction tubes containing the prepared amplification reagent are placed in the reaction tank of the PCR instrument and then stored in the ice bath; b1. The common reverse transcription: reverse transcription at 60 °C for 20 min, then pre-denaturation at 95 °C for 5 min, and 40 cycles of (95 °C 20 s - 60 °C 20 s), i.e., Group (a); b2. The rapid reverse transcription: the heating or cooling rate of the instrument is set to 2 °C/s, 3.5 °C/s or 4.4 °C/s respectively, corresponding to Groups (b)-(d). c. A thermal cycling program is set as follows: pre-denaturation at 95 °C for 5 min, and 40 cycles of (95 °C 20 s, 60 °C 20 s).
   3. Experimental results: As shown in Table 2, when the heating rate of the instrument is 2 °C/s (Group b) and 3.5 °C/s (Group c), there is no significant difference between the detection results of the rapid reverse transcription procedure and the conventional reverse transcription procedure (Group a). When the heating rate of the instrument is 4.4 °C/s (Group d), the detected Ct value is significantly increased, and the template in low copies even could not be detected. However, when the amount of the reverse transcriptase is doubled, even when the heating rate of the instrument is still 4.4 °C/s (Group e), the detected Ct value can return to a level that is not significantly different from that of the conventional reverse transcription procedure (Group a). On one hand, the results of this experiment demonstrate that, under conditions of suitable heating rates and reagents, the detection effect of the rapid reverse transcription method of the present invention is consistent with that of the conventional reverse transcription method.

**Table 2 Ct values of the samples amplified in different methods**

| Detection template | 1 | 2 | 3 | 4 | 5 | 6 |
|---|---|---|---|---|---|---|
| Group a | 30.39 | 34.36 | 37.47 | N | N | N |
| Group b | 30.42 | 34.51 | 37.52 | N | N | N |
| Group c | 30.01 | 34.22 | 38.72 | N | N | N |
| Group d | 31.67 | 35.98 | N | N | N | N |
| Group e | 30.32 | 34.37 | 37.53 | N | N | N |

| | | | | | | |
|---|---|---|---|---|---|---|
| Note: wherein 1 represents 10⁵ copies/ml, 2 represents 10⁴ copies/ml, 3 represents 10³ copies/ml, 4 represents 10² copies/ml, and detection templates 5 and 6 are DEPC water, representing negative controls. "N" indicates that the fluorescent quantitative PCR detection result is negative. | | | | | | |

Although the specific embodiments of the invention have been described in detail, it will be understood by those skilled in the art that various modifications and substitutions may be made to those details in accordance with all the teachings that have been disclosed, all of which are within the protection scope of the present invention. The full scope of the invention is given by the appended claims and any equivalents thereof.

Sequence Listing

<110>  Xiamen University

<120>  RNA REVERSE TRANSCRIPTION AMPLIFICATION METHOD

<130>  IEC170069PEP

<160>  12

<170>  PatentIn Version 3.5

<210>  1
<211>  22
<212>  DNA
<213>  Artificial Sequence

<220>
<223>  Primer

<400>  1
gyttcrgtgc cattcatgtc ac                                                22

<210>  2
<211>  23
<212>  DNA
<213>  Artificial Sequence

<220>
<223>  Primer

<400>  2
gccccatatt caagrtcttt ctc                                               23

<210>  3
<211>  21
<212>  DNA
<213>  Artificial Sequence

<220>
<223>  Primer

<400>  3
agaggatatt ggaccatctg a                                                 21

<210>  4
<211>  19
<212>  DNA
<213>  Artificial Sequence

<220>
<223>  Primer

<400>  4
gaatccatag acacgccag                                                    19

<210>  5
<211>  26
<212>  DNA

<213>  Artificial Sequence

<220>
<223>  Primer

<400>  5
ccgaataata tgatgggcac ttttag                                            26


<210>  6
<211>  24
<212>  DNA
<213>  Artificial Sequence

<220>
<223>  Primer

<400>  6
cctttataat ttgggttggt ctta                                              24


<210>  7
<211>  20
<212>  DNA
<213>  Artificial Sequence

<220>
<223>  Primer

<400>  7
ccagctcaag tgtcagtccc                                                   20


<210>  8
<211>  20
<212>  DNA
<213>  Artificial Sequence

<220>
<223>  Primer

<400>  8
cgcttygatg ctgaattyac                                                   20


<210>  9
<211>  25
<212>  DNA
<213>  Artificial Sequence

<220>
<223>  Primer

<400>  9
gacattgayt tgatgggata tgctc                                             25


<210>  10
<211>  23
<212>  DNA
<213>  Artificial Sequence

<220>

<223>  Primer

<400>  10
gagackagat gtgtgttgaa yca                                               23


<210>  11
<211>  21
<212>  DNA
<213>  Artificial Sequence

<220>
<223>  Primer

<400>  11
cattgcagay atgatcgacc a                                                 21


<210>  12
<211>  26
<212>  DNA
<213>  Artificial Sequence

<220>
<223>  Probe

<400>  12
taygacggrt aycccacrtt yggwga                                            26

## Claims

1. An RNA reverse transcription amplification method comprising steps of reverse transcription of RNA into cDNA and immediate amplification of cDNA, **characterized in that** the process of the reverse transcription of RNA into cDNA is completed under reaction conditions for amplification of cDNA.

2. The RNA reverse transcription amplification method of claim 1, wherein completing the process of the reverse transcription of RNA into cDNA under the reaction conditions for amplification of cDNA means that in the initial stage of cDNA amplification reaction, i.e., during the process where a cDNA amplification reaction system is heated to reach the reaction conditions for amplification of cDNA, the process of the reverse transcription of RNA into cDNA is completed.

3. The RNA reverse transcription amplification method of claim 2, wherein the heating to reach the reaction conditions for amplification of cDNA means that the temperature is raised from 0 °C or above, for example, from 4 °C or above, for example, raised from room temperature.

4. The RNA reverse transcription amplification method of claim 3, wherein the heating to reach the reaction conditions for amplification of cDNA means that the temperature is raised to a thermal denaturation temperature for cDNA amplification.

5. The RNA reverse transcription amplification method of claim 4, wherein the method for amplification of cDNA is a polymerase chain reaction or an isothermal amplification technique.

6. The RNA reverse transcription amplification method of claim 5, wherein the polymerase chain reaction is a polymerase chain reaction performed by thermal cycles of repeated heating and cooling with a heating module or a polymerase chain reaction performed by thermal convection.

7. The RNA reverse transcription amplification method of claim 5, wherein the thermal denaturation temperature is greater than or equal to 90 °C, for example, 91 °C, 92 °C, 93 °C, 94 °C, 95 °C, 96 °C, 97 °C, 98 °C or 99 °C.

8. The RNA reverse transcription amplification method of any one of claims 2 to 7, wherein the heating rate is about 2.0 °C to 4.5 °C/sec, for example, less than or equal to 4.4 °C/sec, or less than or equal to 3.5 °C/sec.

9. The RNA reverse transcription amplification method of any one of claims 1 to 8, wherein the reaction system comprises a reverse transcriptase, preferably, the reverse transcriptase is selected from a murine leukemia reverse transcriptase (MMLV) and an avian myeloblastosis virus reverse transcriptase (AMV).

10. The RNA reverse transcription amplification method of claim 9, wherein the amount of the reverse transcriptase used in the process of reverse transcription of RNA into cDNA is greater than the amount of the reverse transcriptase when the reaction conditions for reverse transcription of RNA into cDNA are separately set.

11. The RNA reverse transcription amplification method of claim 9 or 10, wherein the process of reverse transcription of RNA into cDNA is terminated when the reverse transcriptase is inactivated as the temperature increases during the heating.

12. The RNA reverse transcription amplification method of any one of claims 1 to 11, wherein the RNA fragment is less than 1000 bp in length, for example, less than 900 bp, less than 800 bp, or less than 700 bp in length.
